# EUROPEAN PATENT SPECIFICATION

(11) **EP 2 521 918 B1**
(45) Date of publication and mention of the grant of the patent: **25.03.2015**
(21) Application number: 11700025.7
(22) Date of filing: 07.01.2011
(51) Int. Cl.: G01N 33/68

(54) **DETERMINATION OF EXOSOMAL BIOMARKERS FOR PREDICTING CARDIOVASCULAR EVENTS**
BESTIMMUNG VON BIOMARKER IN EXOSOMEN ZUR VORHERSAGE VON KARDIOVASKULÄREN EREIGNISSEN
DETERMINATION DE LA PRESENCE DE BIOMARQUEURS PRESENTS DANS LES EXOSOMES POUR LA PREDICTION DE RISQUES D'ACCIDENTS CARDIOVASCULAIRES

(30) Priority: 08.01.2010 EP 10150340
(43) Date of publication of application: 14.11.2012
(73) Proprietor: Cavadis B.V., 3511 GC Utrecht (NL)
(72) Inventor: TIMMERS, Leonardus, NL-3572 VA Utrecht (NL); SZE, Siu, Kwan, Singapore 639666 (SG); DE KLEIJN, Dominicus, Paschalis, Victor, NL-3962 ET Wijk bij Duurstede (NL)
(74) Representative: DeltaPatents B.V.
(86) International application number: PCT/EP2011/050171
(87) International publication number: WO 2011/083145

(56) References cited:
- WO-A1-2009/019215
- WO-A2-00/65349
- US-A1- 2007 099 242
- US-A1- 2010 184 046
- ALVAREZ-LLAMAS G. ET AL.: "Recent advances in atherosclerosis-based proteomics: New biomarkers and a future perspective", EXPERT REV. PROTEOMICS, vol. 5, no. 5, October 2008 (2008-10), pages 679-691, XP008114661,
- SIMPSON R.J. ET AL.: "Exosomes: proteomic insights and diagnostic potential", EXPERT REV. PROTEOMICS, vol. 6, no. 3, 1 June 2009 (2009-06-01), pages 267-283, XP009125412,
- LAI R.C. ET AL.: "Exosome secreted by MSC reduces myocardial ischemia/reperfusion injury", STEM CELL RES., vol. 4, no. 3, May 2010 (2010-05), pages 214-222, XP027054731, [retrieved on 2010-01-04]

## Description

### FIELD OF THE INVENTION

The invention relates to the field of risk stratification and/or patient stratification, more particular to the prognosis of risks on cardiovascular events such as stroke, transient ischemic attack (TIA) myocardial infarction (heart attack), cerebral bleeding and other major abnormalities occurring in the blood vessels.

The invention relates in particular to a method of predicting the risk of a subject developing a cardiovascular event.

### BACKGROUND TO THE INVENTION

Established cardiovascular risk factors, including dyslipidemia, smoking, hypertension and diabetes mellitus, have been incorporated into algorithms for cardiovascular risk assessment. However, the identification of patients who are at risk of developing cardiovascular disease remains difficult.

The identification of prognostic biomarkers would be of major added value in recognizing patients who are at risk of suffering future cardiovascular events and who could then be targeted for aggressive preventive measures. For primary cardiovascular events, the prognostic value of biomarkers is very limited since these biomarkers only moderately add to standard risk factors. For secondary events, prognostic biomarkers are non-existent.

The ideal approach in the search for biomarkers is an unbiased approach. Novel molecular techniques such as proteomics opened new possibilities for this purpose.

Recently in the laboratory of the present inventors, this technique was successfully used to discover biomarkers for cardiovascular disease in atherosclerotic plaques. Unfortunately, plaque material can only be obtained through invasive procedures. It is therefore the object of the present invention to provide an alternative method for predicting the risk of a subject developing a cardiovascular event.

Alvarez-Llamas G. et al. : "Recent advances in atherosclerosis-based proteomics: New biomarkers and a future perspective", Expert Rev. Proteomics, vol. 5, no. 5, October 2008, pages 679-691, is a review about atherosclerosis-based proteomics and discloses that exosomes are a new source of biomarkers identification. While referring to cystatin C in the context of classical markers that can help to further characterize patients with cardiovascular diseases, the said document however does not suggest the determination of cystatin C in exosomes.

Simpson R.J. et al. : "Exosomes: proteomic insights and diagnostic potential", Expert Rev. Proteomics, vol. 6, no. 3, 1 June 2009, pages 267-283, is a review about exosomes proteomics and their diagnostic potential. The said document teaches that exosomes are present in body fluids such as urine, amniotic fluid, malignant ascites, bronchoalveolar lavage fluid, synovial fluid, breast milk, saliva and blood. However, it is concerned with the diagnosis of cancer and does not relate to cardiovascular diseases.

### SUMMARY OF THE INVENTION

In the research that led to the invention, proteomic analyses were performed on human plaque and plasma samples. The procedure was hampered, however, by the presence of high-abundant plasma proteins such as albumin and immune-globulins, which complicated the detection of potentially interesting low-abundant proteins. Therefore sub-fractions of plasma were investigated for the presence of proteins that may have predictive value for cardiovascular events.

It was then found that protein constitution in plasma exosome samples from subjects that have suffered a cardiovascular event following the moment of sampling differs from that in patients who have not suffered such a cardiovascular event, and that this difference can be used for prognosis of patients.

Protein secretion out of the cells can occur directly after production (constitutive pathway) or is first stored in the cell and released after a trigger (regulatory pathway). Secretion, however, not only occurs with individual proteins but also occurs via vesicles containing a large number of proteins and RNA. These vesicles are formed with a selection of lipids, protein and RNA from the secreting cell and are released as an intact vesicle. Vesicles in the size of 50-100 nm are called exosomes and the release of exosomes has been described for various cell types, including reticulocytes, B- and T-lymphocytes, dendritic cells, mast cells, platelets, macrophages and alveolar lung cells. In several cell types, including T cells, platelets, dendritic cells and mast cells, secretion of exosomes is regulated by specific stimuli.

While early studies focused on their secretion from diverse cell types *in vitro,* exosomes have now been identified in body fluids such as urine, amniotic fluid, malignant ascites, broncho-alveolar lavage fluid, synovial fluid, breast milk, saliva and blood. Exosomes have a wide range of biological functions, including immune response, antigen presentation, intracellular communication and the transfer of RNA and proteins.

The present inventors found that since exosomes express an array of proteins that reflect the originating host cell, they contain valuable information regarding ongoing (patho)physiologic processes in the human body including information of future cardiovascular events.

This surprising finding now led to the present invention, which thus provides a method for predicting the risk of a cardiovascular event in a patient, based on the detection in plasma exosome samples and/or other micro-vesicles of smaller or larger size from said subject of proteins with prognostic value, herein after referred to as biomarkers or differentially present proteins.

According to the invention, in principle any biomarker with prognostic value may be used. In particular, however, specific markers were identified in plasma exosomes that have predictive value for secondary cardiovascular events.

In one embodiment, the invention thus provides a method of predicting the risk of a subject developing a cardiovascular event comprising detecting a biomarker in an exosome sample or other micro-vesicles of smaller or larger size from said subject, wherein said biomarker comprises at least one protein selected from the group of 6 proteins consisting of: vitronectin (IPI:IPI00298971 SWISSPROT:VTNC_HUMAN,), Serpin F2 (IPI:IPI00879231, SWISSPROT:A2AP_HUMAN), CD14 (IPI:IPI00029260, SWISSPROT:CD14_HUMAN), Cystatin C (IPI:IPI00032293, SWISSPROT:CYTC_HUMAN), Plasminogen (IPI:IPI00019580,SWISSPROT:PLMN_HUMAN) Nidogen 2 (IPI:IPI00028908,SWISSPROT:NID2_HUMAN)

The IPI numbers as disclosed herein between brackets refer to the International Protein Index (http://www.ebi.ac.uk/IPI), as indexed on December 4, 2010 followed by Swissprot database Entry name as indexed on November 30, 2010. The referenced index numbers (database accessions) as used herein include reference to fragments, isoforms and modifications thereof, hence the present invention foresees the use of fragments of the proteins as well as modifications and derivatives of the proteins disclosed herein as biomarkers in the context of the various aspects of the present invention.

According to the invention a biomarker comprises one protein or a set of multiple proteins. Such a biomarker is also identified herein as a profile or protein profile. A profile may comprise 1, 2, or more than 2 such as 3, 4, 5, 6 of the proteins Vitronectin (IPI:IPI00298971 SWISSPROT:VTNC_HUMAN,), Serpin F2 (IPI:IPI00879231, SWISSPROT:A2AP_HUMAN), CD14 (IPI:IPI00029260, SWISSPROT:CD14_HUMAN), Cystatin C (IPI:IPI00032293, SWISSPROT:CYTC_HUMAN), Plasminogen (IPI:IPI00019580,SWISSPROT:PLMN_HUMAN), Nidogen 2 (IPI:IPI00028908, SWISSPROT:NID2_HUMAN). According to the invention a profile may be used that comprises any number and any combination of these proteins.

The skilled person will understand that instead of detecting the complete biomarker protein, one may detect peptide fragments of said biomarker proteins which are derived from the biomarker proteins by fragmentation thereof. The term peptide fragment as used herein refers to peptides having between 5 and 50 amino acids. These peptide fragments preferably provide a unique amino acid sequence of the protein, and are associated with the cardiovascular events as disclosed herein.

The proteins and/or peptide fragment may optionally be detected as chemically modified proteins and/or peptides, such chemical modification may for instance be selected from the group consisting of glycosylation, oxidation, (permanent) phosphorylation, reduction, myristylation, sulfation, acylation, acetylation, ADP-ribosylation, amidation, hydroxylation, iodination, and methylation. A large number of possible protein modifications is described in the RESID database at http://www.ebi.ac.uk/RESID (release December 2 2010) (Garavelli, J.S. (2004) The RESID Database of Protein Modifications as a resource and annotation tool; Proteomics 4: 1527-1533) and in Farriol-Mathis, N., Garavelli, J.S., Boeckmann, B., Duvaud, S., Gasteiger, E., Gateau, A., Veuthey, A., Bairoch, A. (2004) Annotation of post-translational modifications in the Swiss-Prot knowledge base. Proteomics 4(6): 1537-50. The skilled artisan is well aware of these modifications.

The biomarker protein or a peptide fragment thereof is detected in exosomes or other vesicles somewhat larger or smaller in size that are preferably found in body fluids like serum, plasma or blood. Alternatively, exosomes or such other vesicles from other body fluids such as urine, amniotic fluid, malignant ascites, bronchoalveolar lavage fluid, synovial fluid, breast milk, saliva can be used.

In the application the word "exosome" is intended to include other vesicles that are smaller than about 50 nm or larger than 100 nm but still fall within the range of about 20 to about 500 nm.

According to the present invention, the cardiovascular event to be predicted is preferably selected from the following conditions: vascular death or sudden death, fatal or non fatal stroke, fatal or non fatal myocardial infarction, fatal or non fatal rupture of an abdominal aortic aneurysm, rupture of abdominal aortic aneurysm confirmed by laparatomy, vascular intervention, coronary artery disease, transient ischemic attack (TIA), peripheral arterial disease, acute coronary syndrome, heart failure or re-stenosis of carotid, coronary, femoral or other arteries.

The method of the present invention may suitably be used for risk stratification and/or patient selection (such as for clinical trials), for monitoring of disease, and the markers may be used as clinical biomarkers for safety and efficacy studies (e.g. as surrogate endpoint markers).

The cardiovascular event may be a primary event in a subject that has not yet suffered a cardiovascular event but is in particular a secondary event occurring in a subject already having suffered such an event before. According to the invention it is possible to discriminate between patients that already had a cardiovascular event and are at risk of suffering an additional event and patients who had such an event and do not have an increased risk of suffering a further event.

Suitably, the prognosis is made by using exosomes as the sample and preferably the biomarker comprising Vitronectin, Serpin F2, CD14, Cystatin C, Plasminogen, Nidogen 2 or any combination thereof as the protein(s) to be detected.

The present invention will be further illustrated in the Examples that follow and that are not intended to limit the invention in anyway. Reference is made to the following figures:

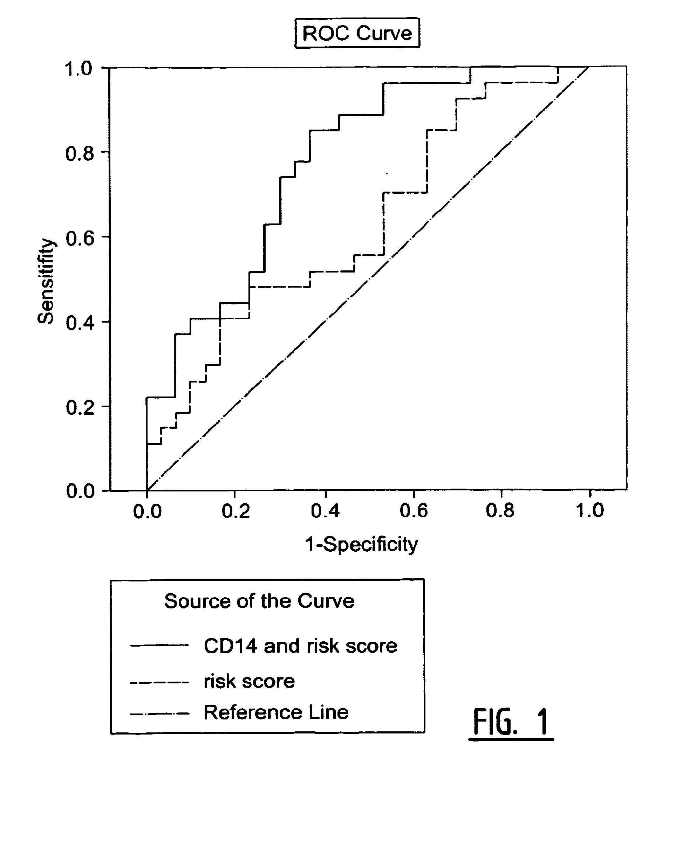

**Figure 1****:** the graph shows two ROC analyses for CD14. The solid black line is the ROC analysis for the CD14 + risk factors (AUC=0.778, *P* = 0.001); the broken black line is the ROC analysis for the risk factors alone (AUC=0.630, *P* = 0.093). The solid grey line is the reference line and represents an AUC of 0.5 (that is, no discrimination).

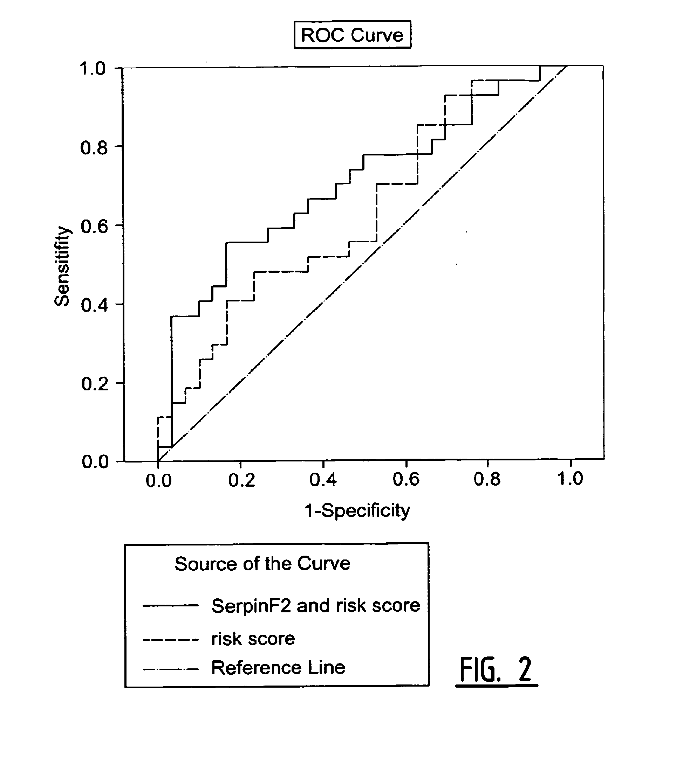

**Figure 2****:** the graph shows two ROC analyses for **Serpin F2.** The solid black line is the ROC analysis for the SerpinF2 + risk factors (AUC=0.701, *P* = 0.009); the broken black line is the ROC analysis for the risk factors alone (AUC=0.630, *P* = 0.093). The solid grey line is the reference line and represents an AUC of 0.5 (that is, no discrimination).

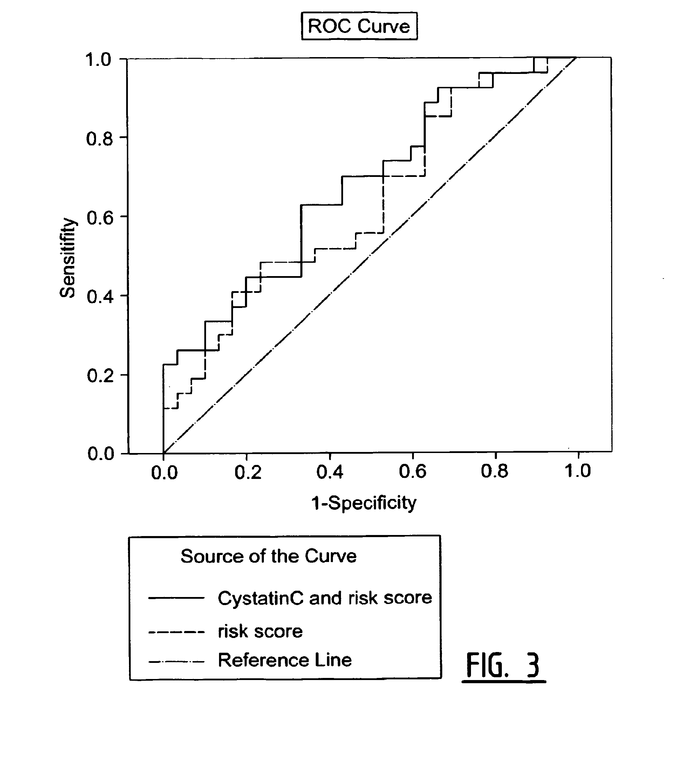

**Figure 3****:** the graph shows two ROC analyses for **CystatinC.** The solid black line is the ROC analysis for the CystatinC +risk factors (AUC=0.677, *P* = 0.022); the broken black line is the ROC analysis for the risk factors alone (AUC=0.630, *P* = 0.093). The solid grey line is the reference line and represents an AUC of 0.5 (that is, no discrimination).

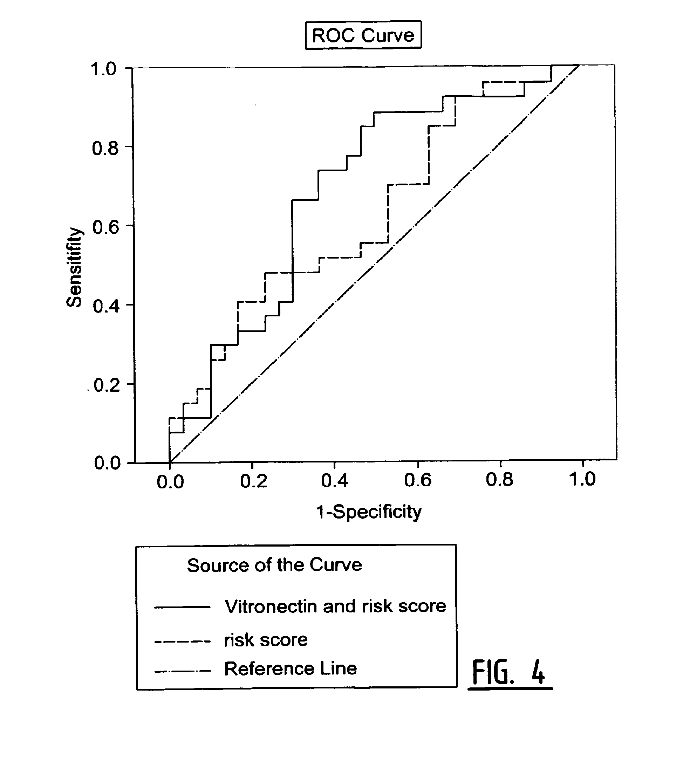

**Figure** 4: the graph shows two ROC analyses for **Vitronectin.** The solid black line is the ROC analysis for the Vitronectin + risk factors (AUC=0.690, *P* = 0.014); the broken black line is the ROC analysis for the risk factors alone (AUC=0.630, *P* = 0.093). The solid grey line is the reference line and represents an AUC of 0.5 (that is, no discrimination).

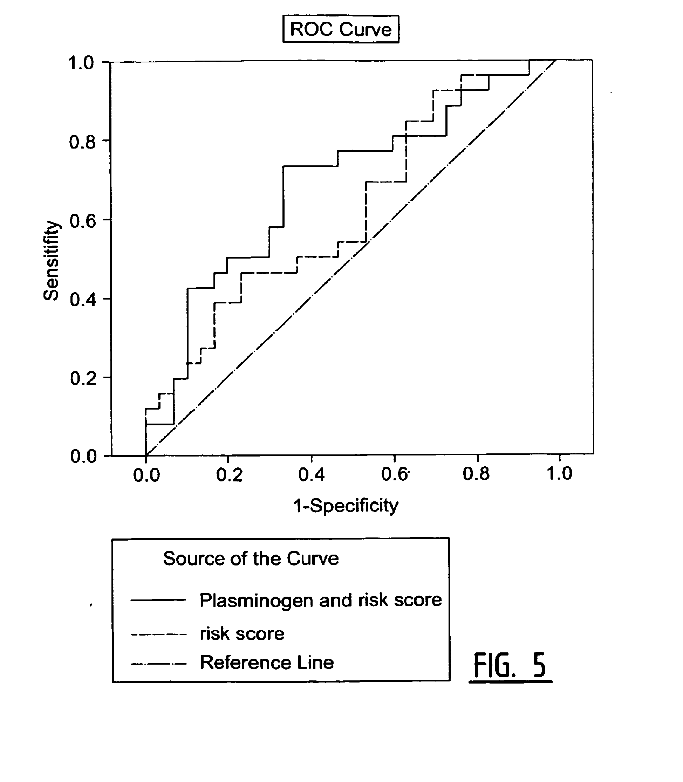

**Figure 5****:** the graph shows two ROC analyses for **Plasminogen.** The solid black line is the ROC analysis for the Plasminogen + risk factors (AUC=0.654, *P* = 0.046); the broken black line is the ROC analysis for the risk factors alone (AUC=0.630, *P* = 0.093). The solid grey line is the reference line and represents an AUC of 0.5 (that is, no discrimination).

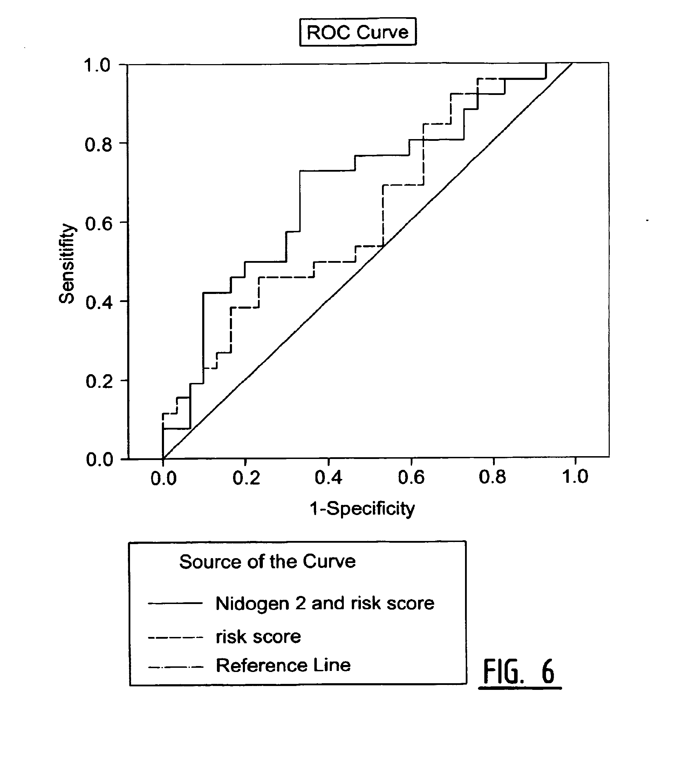

**Figure 6****:** the graph shows two ROC analyses for **Nidogen 2.** The solid black line is the ROC analysis for the **Nidogen 2** + risk factors (AUC=0.686, *P* = 0.017); the broken black line is the ROC analysis for the risk re alone (AUC=0.630, *P* = 0.093). The solid grey line is the reference line and represents an AUC of 0.5 (that is, no discrimination).

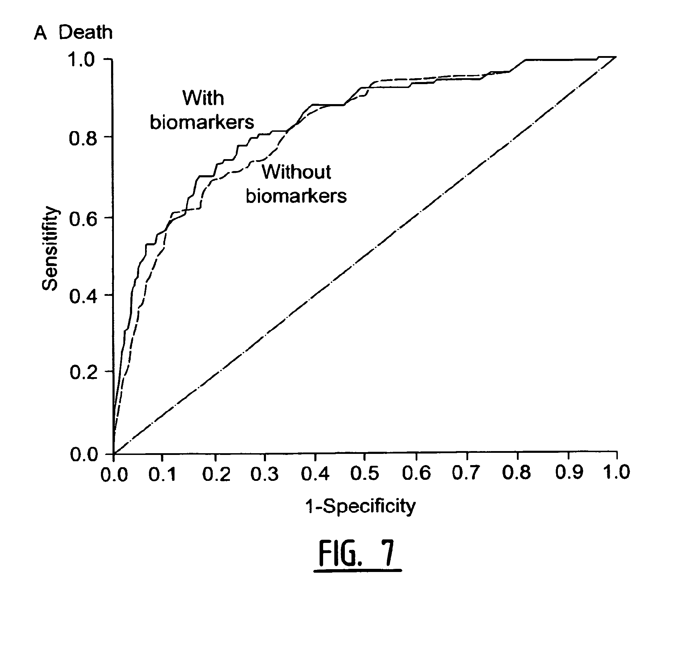

**Figure 7****:** a published ROC curve.

### EXAMPLES

### EXAMPLE 1

### Quantitative Proteomics on human plasma exosomes with follow-up

### Study population and design

The Athero-Express is a longitudinal vascular biobank study, which includes biomaterials from patients undergoing carotid and femoral end-arterectomy in two Dutch hospitals (UMC Utrecht and St. Antonius Hospital Nieuwegein). About 2000 patients have been included thus far. Plasma and tissue samples were obtained from all patients before (blood) or during end-arterectomy.

All patients underwent clinical follow-up 1 year after surgical intervention and filled in postal questionnaires 1, 2 and 3 years after the operation. When patients did not respond to the questionnaire, the general practitioner was contacted by phone. Adjudication of the outcome events was done by an independent outcome event committee that was blinded to laboratory results. Two members of the committee independently assessed all endpoints. In case of disagreement, a third opinion was obtained.

### The exosomal proteome

Plasma samples from 50 patients that suffered a coronary event during follow up and from 50 age and sex matched control patients, without a secondary event during follow up, were pooled separately and exosomes were isolated by filter separation and ultracentrifugation. Quantitative proteomics were used to identify the exosomal protein content, and allowed to compare the expression levels of the proteomes from patients that suffered events during follow up with the proteomes of control patients.

Exosomes were isolated from frozen human plasma by filter separation followed by ultracentrifugation (cf. Marie-Pierre Caby et al. Exosomal-like vesicles are present in human blood plasma; International Immunology, Vol. 17, No. 7, pp. 879-887).

Typical exosomal proteins such as CD9 and CD81 were detected in the exosome pellet using western blotting. FACS analysis with beads of defined sizes demonstrated that the pellet contains mostly particles of 50-100 nm which is in accordance with the size of exosomes.

### Protein extraction and digestion

The exosome pellets collected in the Athero-Express biobank plasma were after ultracentrifugation dissolved in 40 µl 6% SDS in HPLC pure water. Plaque protein was, after grinding the plaque material without any blood remains to powder, also extracted with 6% SDS. Digestion and subsequent labeling, HPLC separation and mass spectrometry analysis was identical for plaque and exosome proteins. The protein content was determined by 2-D Quant Kits. After protein reduction and alkylation, the protein mixture was diluted 20 times with 50 mM triethylammonium bicarbonate (TEAB) and protein digestion was initiated by adding trypsin in a 1:40 trypsin-to-protein ratio. The protein digests were desalted using a Sep-Pak C18 cartridge and dried in a Speedvac.

These digests were labeled with iTRAQ reagents according to the manufacturer's protocol. Briefly, digested proteins were reconstituted in 30 µCl of dissociation buffer and mixed with 70 µl of ethanol-suspended iTRAQ reagents (one iTRAQ reporter tag per protein sample, mass tag 114-117 Dalton). Labeling reactions were carried out at RT for 1 hr before all the samples were mixed into a single tube and dried using a Speedvac.

### Strong Cation Exchange fractionation

The combined iTRAQ labeled samples were reconstituted with 200 µl buffer A (10 mM KH₂PO₄, pH 3.0, 25% v/v acetonitrile), and loaded onto a PolySULFOETHYL A column (200 mm length x 4.6 mm ID, 200-Å pore size, 5 µm particle size) on a Shimadzu prominence HPLC system. The sample was fractionated using a gradient of 100% buffer A for 5 min, 5-30% buffer B (10 mM KH₂PO₄, pH 3.0, 500 mM KCl and 25% v/v acetonitrile) for 40 min, 30-100% buffer B for 5 min, and finally 100% buffer B for 5 min, at a constant flow rate of 1 ml/min for a total of 60 min. The eluted fractions were monitored through a UV detector at 214 nm wavelength.

Fractions were collected at 1-min intervals and consecutive fractions with low peak intensity were combined. Finally, a total of 20 fractions was obtained and dried in a Speedvac. Each fraction was reconstituted in 0.1% trifluoroacetic acid and desalted. Desalted samples were dried in a Speedvac and stored at -20°C prior to mass spectrometric analysis.

### Mass spectrometric analysis using Q-STAR

The dried fraction was reconstituted in 100 µl of 0.1% formic acid. Each sample was analyzed two times using a Q-Star Elite mass spectrometer, coupled to an online Shimadzu prominence HPLC system. For each analysis, 50 µl of peptide mixture was injected and separated on a home-packed nanobored C18 column with a picofrit nanospray tip (75 µm ID x 15 cm, 5 µm particles). The separation was performed at a flow rate of 20 µl/min with a splitter of a 90 min gradient. The mass spectrometer was set to perform data acquisition in the positive ion mode, with a selected mass range of 300-2000 *m*/*z.* Peptides with +2 to +4 charge states were selected for MS/MS and the time of summation of MS/MS events was set to 2 s. The three most abundantly charged peptides above a 5 count threshold were selected for MS/MS and dynamically excluded for 30 s with ±30 mmu mass tolerance.

Peptide quantification and protein identification were performed using ProteinPilot software v2.0.1 by searching the combined data from the 2 runs against the International Protein Index (IPI) human database (indexed December 19, 2009). The Paragon algorithm in ProteinPilot software was used whereby trypsin was selected as the digestion agent and cysteine modification of methylethanethiosulfonate.

All proteins reported had an expectation value of less than 0.05 (unused score 1.3).

Quantitative proteomics were performed on exosomes from 50 patients that suffered a coronary event during follow up (Group 1) and from 50 matched control patients that did not suffer a secondary event during follow up (Group 2). Each group was run twice in the same iTraq experiment revealing data of 2 events proteomes and 2 control proteomes.

2148 different proteins were identified in the samples, including a large number of proteins identified earlier in exosome proteomics such as CD9, CD81, Annexins, Clathrin heavy chain, Enolase 1 and many more (Olver C, Vidal M. Proteomic analysis of secreted exosomes. Subcell Biochem. 2007;43:99-131).

### Results of exosome proteomics

Group 1 and 2 were then compared using the quantitative iTRAQ data. Quantitative data were available from 2 pooled events samples (Group 1 in duplo) and 2 pooled control samples (Group 2 in duplo). Based on pilots, it was determined that a ratio of 1.2 and above means that there is significantly higher level of the protein in the event while a ratio of 0.8 and lower is a significant lower level in the event. First selection was based on proteins with identical duplo's (both below 0.8, both above 1.2 or both between 0.8 and 1.2).

Second selection was based on proteins with lower (events/controls <0.8) or higher (events/controls >1.2) expression in group 1 vs. group 2. This-revealed a list of 116 proteins.

This list of 116 proteins was uploaded and analyzed in Ingenuity Pathway Analysis software (version 8.0). From the 116 proteins, 102 proteins were in the Ingenuity database. This revealed that the 102 proteins are different types of proteins, including transmembrane receptors, transporters and transcription regulators, proteins that are not present in plasma. Ingenuity analysis also showed that 3 canonical pathways are significantly overrepresented in these 102 proteins:
1. Acute Phase Response Signaling (p= 3.5*10⁻¹¹)
2. Coagulation System (p= 3.6*10⁻¹¹)
3. Atherosclerosis Signaling (p= 3* 10⁻⁴)

Subsequently, this group of 102 differentially expressed proteins was complemented with a selection of plaque material derived proteins and finally narrowed drown to a combined set of 34 selected exosome- and plaque-derived proteins for further validation in exosome samples of individual patient samples.

### Results of plaque protein proteomics

Using the Athero-Express cohort, 40 carotid end-arterectomy patients were selected of which 20 had a secondary cardiovascular event during follow-up and 20 (age, sex and time to follow-up matched) controls that did not suffer from a secondary event during follow-up.

Quantitative proteomics was performed on plaque samples as for the exosome proteomics. However, since 40 individual plaques were analyzed, four plaque extracts were run simultaneously each differently labeled by the iTraq reagent (114, 115, 116, 117 resp.). Each run consisted of two plaque extracts of patients that suffered a cardiovascular event and for each patient a sex and age matched control, so in total four plaque extracts in two pairs of event and control.

After the search, an excel file was generated containing the protein ID and the relative value of the two event/control pairs for each of the protein IDs.

Analysis was performed after 10 runs including 20 pairs of events with matched controls with a total of 40 patients. Using Excel 2007 with the Merge Table Add-in, a total list of protein IDs was generated. Normalization between the different runs occurred via total peptide area correction.

Statistical analysis comparing events with controls using a Mann-Whitney test revealed 264 proteins that were significantly different (p<0.05) between events and controls in plaque.

### Selection of exosome and plaque-derived proteins

The plaque is the origin of atherosclerotic disease leading to cardiovascular events. For this, it is very likely that plaque proteins related to future cardiovascular events can also be found in exosomes especially the plaque proteins that are related to the pathways over-represented in exosome proteins that differ between cardiovascular events and controls.

Having established that 3 canonical pathways (acute phase, coagulation and atherosclerosis) are over-represented in exosomes, the 264 protein data-set with differentially expressed plaque proteins between events and controls was investigated in 2 ways.

Selection was based on the presence of proteins that are related to the 3 atherosclerosis related canonical pathways and for which 2 antibodies and a recombinant protein were available.

Also from the 112 exosome-derived proteins, markers were selected based on over-representation of 3 atherosclerosis related canonical pathways and the availability of 2 antibodies and a recombinant protein. From the selected plaque and exosome proteins for which antibodies and recombinant protein were available, 34 proteins were chosen for Luminex bead assay development.

For 17 proteins out of those 34 proteins, a reproducible and quantitative Luminex bead assay was set up that could be used for measuring the protein content in exosomes isolated from individual serum samples.

### EXAMPLE 2

### Verification of the selected proteins in a proof of concept study in blood samples of individual Athero-Express patients Study Objective

The objective of this study was to identify in blood samples of individual patients which of those 17 biomarkers were differentially expressed between patients suffering from a secondary coronary event and healthy controls.

### Study design

Patients in this study underwent surgery of the carotid arteries because of a primary cerebral-vascular event i.e. a stroke or Transient Ischemic Attack (TIA) and were followed-up for three years. The 17 markers were measured in blood samples of patients who suffered from a secondary coronary event (29 samples) and age and sex matched controls (30 samples). The secondary coronary events were defined as myocardial infarction (fatal and non-fatal), cardiovascular death, sudden death, coronary angioplasty, and coronary artery bypass graft (CABG).

### Materials and methods

Exosomes were isolated from the plasma using the ultracentrifugation technique. Proteins extracted from the exosome samples were measured in multiplex Luminex bead assays.

### Statistical analyses

Statistical analyses were performed using the statistical software package PASW Statistics 17.0.2 (SPSS Inc, Chicago, Illinois). Discrimination (a measure of how well the model can separate events and controls) is most often measured by the area under the receiver operating characteristic (ROC) curve, an established method for assessing biomarkers (Hlatky et al.American Heart Association Expert Panel on Subclinical Atherosclerotic Diseases and Emerging Risk Factors and the Stroke Council. Criteria for evaluation of novel markers of cardiovascular risk: a scientific statement from the American Heart Association. Circulation. 2009 May 5;119(17):2408-16).

ROC analyses were performed to determine the ability of the marker, in conjunction with a risk score, to distinguish between patients with and without coronary future events.

In an ROC analysis, the specificity and sensitivity of a specific test is given for different cut-off values of the test outcome. **Figure 7** depicts a published ROC curve. The ideal test, with optimal sensitivity and specificity will follow a curve with 1 - specificity = 0 (the y-axis) and then with sensitivity = 1.0 (see bold line). A test without any value will follow the black straight line. The discriminative power of the test is provided as "area under the curve" (AUC). AUC values range between 0.5 (no discrimination) and 1.0 (perfect discrimination).

Statistical significance was set at *P* = 0.05. The risk score was based on 7 traditional cardiovascular risk factors (gender, age, cholesterol, systolic blood pressure, smoking status, history of peripheral artery disease, and history of coronary artery disease).

### Results

When a new test for prediction of disease is evaluated it has to be compared with the risk predictors that are already available in the clinical arena. In this case these are the traditional risk factors (see above under the section "Statistical analyses"). Therefore the AUC of the traditional risk factors alone were compared with the AUC of the traditional risk factors + the new biomarker. Thus an increase in AUC is explained by the new biomarker.

The AUC of the risk score alone was found to be 0.630 (*P* = 0.093). Six of the 18 markers, assessed in conjunction with the risk score, showed an increase in the AUC: CD14 0.778 (*P* = 0.001) (**Figure 1**); Serpin F2 0.701 (*P* = 0.009) (**Figure 2**); Cystatin C 0.677 (*P* = 0.022) (**Figure 3**); Vitronectin 0.690 (*P* = 0.014) (**Figure 4**); Plasminogen 0.654 (*P* = 0.046) (**Figure 5**); and Nidogen 2 0.686 (*P* = 0.017) (**Figure 6**).

These six proteins are thus in particular useful as a biomarker in exosomes as the sample to be tested in order to allow a reliable prognosis of a patient suffering a future cardiovascular event.

## Claims

1. In vitro Method of predicting the risk of a subject developing a cardiovascular event, comprising determining the presence of a biomarker that is indicative of the risk of developing a cardiovascular event in exosomes from the subject, wherein the biomarker is a protein selected from the group consisting of: Vitronectin, SerpinF2, CD14, Cystatin C, Plasminogen and Nidogen 2.

2. Method as claimed in claim 1, wherein the exosomes are isolated from a body fluid selected from the group consisting of: serum, plasma, blood, urine, amniotic fluid, malignant ascites, bronchoalveolar lavage fluid, synovial fluid, breast milk and saliva.

3. Method as claimed in claim 1 or 2, wherein the biomarker is any combination of two or more proteins selected from the group consisting of: Vitronectin, SerpinF2, CD14, Cystatin C, Plasminogen and Nidogen 2.

4. Method as claimed in any one of claims 1-3, wherein the cardiovascular event is selected from the group consisting of: vascular death or sudden death, fatal or non fatal stroke, fatal or non fatal myocardial infarction, fatal or non fatal rupture of an abdominal aortic aneurysm, rupture of abdominal aortic aneurysm confirmed by laparatomy, vascular intervention, coronary artery disease, transient ischemic attack (TIA), peripheral artherial disease, acute coronary syndrome, heart failure or restenosis of carotid, coronary femoral or other arteries.

## Patentansprüche

1. In vitro-Verfahren zur Vorhersage des Risikos, dass ein Subjekt ein Herz-Kreislauf-Ereignis entwickelt, umfassend die Bestimmung der Anwesenheit eines biologischen Markers, der das Risiko der Entwicklung eines Herz-Kreislauf-Ereignisses anzeigt in Exosomen vom Subjekt, wobei der biologische Marker ein Protein ist, ausgewählt aus der Gruppe bestehend aus: Vitronektin, SerpinF2, CD14, Cystatin-C, Plasminogen und Nidogen 2.

2. Verfahren nach Anspruch 1, wobei die Exosomen von einer Köperflüssigkeit isoliert sind, ausgewählt aus der Gruppe bestehend aus: Serum, Plasma, Blut, Urin, Fruchtwasser, maligner Aszites, durch bronchoalveoläre Lavage gewonnene Flüssigkeit, Synovialflüssigkeit, Muttermilch und Speichel.

3. Verfahren nach Anspruch 1 oder 2, wobei der biologische Marker jede Kombination aus zwei oder mehreren Proteinen ist, ausgewählt aus der Gruppe bestehend aus: Vitronektin, SerpinF2, CD14, Cystatin-C, Plasminogen und Nidogen 2.

4. Verfahren nach einem der Ansprüche 1 bis 3, wobei das Herz-Kreislauf-Ereignis ausgewählt ist aus der Gruppe bestehend aus: vaskulärem Tod oder plötzlichem Tod, tödlichem oder nicht-tödlichem Schlaganfall, tödlichem oder nicht-tödlichem Myokardinfarkt, tödlicher oder nicht-tödlicher Ruptur eines Bauchaortenaneurysmas, Ruptur eines Bauchaortenaneurysmas bestätigt durch Laparatomie, Gefäßeingriff, Erkrankung der Koronararterien, transientem ischämischem Anfall (TIA), peripherer arterieller Verschlusskrankheit, akutem Koronarsyndrom, Herzversagen oder Restenose der Halsschlagader, des Herzkranzgefässes, der Femoral- oder anderer Arterien.

## Revendications

1. Procédé *in vitro* permettant de prédire le risque de développement par un patient d'un événement cardio-vasculaire, comprenant la détermination de la présence d'un biomarqueur qui indique le risque de développement d'un événement cardio-vasculaire dans des exosomes prélevés sur le patient, dans lequel le biomarqueur est une protéine choisie dans le groupe constitué par : la vitronectine, la serpine F2, CD14, la cystatine C, le plasminogène et le nidogène 2.

2. Procédé selon la revendication 1, dans lequel les exosomes sont isolés à partir d'un fluide corporel choisi dans le groupe constitué par : le sérum, le plasma, le sang, l'urine, le fluide amniotique, des ascites malignes, un fluide de lavage broncho-alvéolaire, un fluide synovial, le lait maternel et la salive.

3. Procédé selon la revendication 1 ou 2, dans lequel le biomarqueur est toute combinaison de deux ou plus de deux protéines choisies dans le groupe constitué par : la vitronectine, la serpine F2, CD14, la cystatine C, le plasminogène et le nidogène 2.

4. Procédé selon l'une quelconque des revendications 1 à 3, dans lequel l'événement cardio-vasculaire est choisi dans le groupe constitué par : un décès d'origine vasculaire ou une mort subite, un accident cérébral vasculaire fatal ou non fatal, un infarctus du myocarde fatal ou non fatal, la rupture fatale ou non fatale d'un anévrisme aortique abdominal, la rupture d'un anévrisme aortique abdominal confirmé par laparotomie, une intervention vasculaire, une maladie coronarienne, un accident ischémique transitoire (AIT), une maladie des artères périphériques, un syndrome coronarien aigu, une insuffisance cardiaque ou une resténose de la carotide, de l'artère coronaire, de l'artère fémorale ou d'autres artères.
